# FASCICULE DE BREVET EUROPEEN

(11) **EP 3 958 993 B1**
(45) Date de publication et mention de la délivrance du brevet: **23.07.2025**
(21) Numéro de dépôt: 20720747.3
(22) Date de dépôt: 06.04.2020
(51) Int. Cl.: B01D 11/00, C07C 1/207, C07C 29/86, C07C 45/80, C07C 31/08, C07C 47/06, C07C 11/167, B01D 11/04

(54) **PROCEDE DE PURIFICATION D'UNE CHARGE HYDROALCOOLIQUE COMPRENANT DE L'ETHANOL, DE L'ACETALDEHYDE**
VERFAHREN ZUR REINIGUNG EINES WÄSSRIG-ALKOHOLISCHEN AUSGANGSSTOFFES MIT ETHANOL UND ACETALDEHYD
METHOD FOR PURIFYING AN AQUEOUS-ALCOHOLIC FEEDSTOCK COMPRISING ETHANOL AND ACETALDEHYDE

(30) Priorité: 25.04.2019 FR 1904345
(43) Date de publication de la demande: 02.03.2022
(73) Titulaire: IFP Energies nouvelles, 92852 Rueil-Malmaison Cedex (FR); COMPAGNIE GENERALE DES ETABLISSEMENTS MICHELIN, 63000 Clermont-Ferrand (FR)
(72) Inventeur: AUGIER, Frederic, 92852 Rueil-Malmaison Cedex (FR); DREGER, Pierre Olivier, 92852 Rueil-Malmaison Cedex (FR)
(74) Mandataire: IFP Energies nouvelles
(86) Numéro de dépôt international: PCT/EP2020/059739
(87) Numéro de publication internationale: WO 2020/216603

(56) Documents cités:
- WO-A1-2017/194559
- WO-A1-2018/001982
- WO-A1-2020/064317
- FR-A1- 3 057 467

## Description

### Domaine technique

La présente invention concerne un procédé de traitement d'une charge comprenant au moins de l'eau, de l'éthanol, de l'acétaldéhyde, par extraction et contre-extraction liquide-liquide, permettant de maximiser l'élimination des impuretés, en particulier peu ou non polaires, tout en optimisant la récupération de l'éthanol et de l'acétaldéhyde.

Le procédé selon l'invention peut avantageusement être intégré dans un procédé plus global de conversion de l'éthanol en butadiène, appelé encore procédé Lebedev. Il permet alors de purifier l'effluent liquide issu des réacteurs de conversion tout en améliorant la récupération de l'éthanol et de l'acétaldéhyde non convertis en butadiène.

### Technique antérieure

Le procédé de production de butadiène à partir d'éthanol a été développé, en particulier, par des équipes américaines durant la seconde guerre mondiale à partir des travaux d'Ostromilenski.

Dans ce procédé, la conversion par passe est inférieure à 50%, ce qui implique des recyclages importants de l'éthanol et de l'acétaldéhyde. De plus, une grande variété d'impuretés de différentes natures (hydrocarbures saturés, insaturés, aromatiques, produits oxygénés tels que alcools, cétones, aldéhydes, phénols, acides, esters, éthers) et ayant des masses molaires très différentes est produite (entre 50 et 10 000 g/mol).

Il est donc nécessaire de mettre en place un enchaînement d'opérations unitaires dans le but d'éliminer le maximum d'impuretés en perdant le moins possible d'éthanol et d'acétaldéhyde. D'un point de vue économique, il est primordial de diminuer le coût de production du butadiène, ce qui nécessite de :
a) perdre le moins possible d'éthanol et d'acétaldéhyde
b) ne pas recycler d'impuretés dans les réacteurs qui induiraient une chute de sélectivité en butadiène, ou qui s'accumuleraient à des niveaux non acceptables, nécessitant une purge et donc des pertes en éthanol et acétaldéhyde.

À la sortie des réacteurs catalytiques, l'effluent produit, comprenant du butadiène, de l'éthanol, de l'eau, de l'acétaldéhyde et des impuretés, subit plusieurs opérations unitaires afin de séparer les sous-produits gazeux et liquides non désirés du butadiène formé et des composés éthanol, eau, acétaldéhyde, appelés composés « nobles », liquides et gazeux étant entendus à température et pression ambiante.

Parmi les sous-produits gazeux à température et pression ambiante, on peut citer l'hydrogène, le monoxyde de carbone, le dioxyde de carbone, les alcanes et les oléfines en C₁-C₄. Il est primordial d'éliminer ces sous-produits de l'effluent riche en butadiène afin d'obtenir un produit butadiène aux spécifications requises. Parmi les sous-produits liquides à température et pression ambiante, on peut citer l'acétone, le diéthyléther, le butanal, le butanol, le butanone, l'éthyle acétate, le crotonaldéhyde et l'acide acétique. D'autres sous-produits peuvent être générés en plus faible quantité dans la zone réactionnelle. Dans la suite du document, le terme « impuretés » désignera cet ensemble de milliers de composés hydrocarbonés ou oxygénés.

Dans les premiers schémas de procédé des équipes américaines, l'éthanol, l'acétaldéhyde, l'eau et les sous-produits liquides sont séparés par un enchaînement de trois colonnes à distiller (brevet US 2,403,742). L'effluent riche en éthanol, acétaldéhyde, eau et sous-produits liquides alimente une première colonne à distiller dans laquelle un effluent riche en acétaldéhyde est séparé du reste de l'effluent. Une seconde colonne à distiller permet de séparer les sous-produits liquides d'un effluent riche en éthanol et eau. La dernière colonne à distiller permet de séparer l'éthanol de l'eau. La majeure partie des brevets de procédé déposés dans la période 1940-1960 par les sociétés Carbide & Carbon ou Koppers (US 2,403,743 ; US 2,393,381 ; US 2,395,057 et US 2,439,587) visent à améliorer cette partie du schéma.

Une des problématiques du procédé, observées dans les années 1945, est une formation importante de diéthylacétal et/ou hémiéthylacétal résultant notamment de la réaction de l'éthanol avec l'acétaldéhyde, ce qui entraine une perte non négligeable en réactifs (éthanol, acétaldéhyde) et donc une baisse du rendement en butadiène. Toussaint et al., Industrial and Engineering Chemistry; 1947; Vol. 39, No. 2, p. 120-125, indiquent en particulier que 20 kg de diéthylacétal sont produits pour une tonne de butadiène formé.

Dans les brevets FR 3 026 100 et FR 3 026 101, l'élimination des impuretés liquides se fait au moins en partie par extraction liquide-liquide. L'effluent liquide en sortie des réacteurs, comprenant de l'éthanol, de l'acétaldéhyde, de l'eau et des impuretés, alimente une colonne d'extraction liquide-liquide. Cette dernière est alimentée en fond par un solvant de lavage qui a pour but de laver à contre-courant la charge. A la sortie de cette section de lavage, l'extrait est composé majoritairement du solvant de lavage et des sous-produits extraits (comme le diéthyléther) et comprend de faibles quantités d'éthanol et d'acétaldéhyde. Cet extrait est ensuite lavé à l'eau dans le but de ré-extraire l'éthanol et l'acétaldéhyde et ainsi minimiser les pertes en éthanol et acétaldéhyde. Le solvant de lavage employé pour cette opération unitaire est constitué d'un mélange d'hydrocarbures ayant entre 6 et 40 atomes de carbone.

Dans cette configuration, une forte proportion du diéthylacétal et hémiéthylacétal, formés en amont de l'étape d'extraction liquide-liquide, notamment par réaction de l'éthanol et de l'acétaldéhyde non convertis présents dans l'effluent liquide en sortie de réacteurs, sont extraits par le solvant de lavage. Cela a pour conséquence d'entrainer une perte en équivalent éthanol et acétaldéhyde non négligeable et donc augmenter le prix de production du butadiène.

Le brevet FR 3 057 467 propose quant à lui un procédé de purification de l'effluent liquide comprenant l'éthanol, l'eau et l'acétaldéhyde, du procédé Lebedev décrit notamment dans le brevet FR 3 026 100 ou FR 3 026 101, par extraction et contre-extraction liquide-liquide avec décomposition du diéthylacétal catalysée par la présence d'un acide dans le solvant aqueux de contre-extraction lors de l'étape de contre-extraction. Cependant, un tel procédé avec la mise en œuvre d'un acide dissout dans la phase aqueuse entraine une consommation et donc une perte plus ou moins importante de molécules acide introduites dans le solvant de contre-extraction.

La demande WO 2017/194559 A1 concerne également un procédé pour éliminer des impuretés dans un mélange aqueux d'éthanol et d'acétaldéhyde, par extraction et réextraction liquide-liquide. Cependant, ce procédé nécessite l'utilisation d'un solvant donneur de Lewis, un ajustement du débit massique de l'effluent riche en solvant donneur de Lewis dans l'étape d'extraction et un ajustement du débit massique de solvant auxiliaire employé dans l'étape de réextraction, de manière à extraire le maximum d'impuretés tout en limitant les pertes en éthanol et acétaldéhyde.

La demande WO 2018/001982 A1 se rapporte à un procédé de production de butadiène à partir d'éthanol, comprenant une étape de purification du butadiène basée sur des distillations extractives successives et une étape (E) de traitements des effluents impuretés comprenant de l'eau, de l'éthanol et de l'acétaldéhyde, ladite étape (E) mettant en oeuvre une section de lavage - contre lavage. Si le contre-lavage utilise l'eau comme solvant de contre-extraction, la demande WO 2018/001982 A1 ne mentionne pas de moyen pour limiter les pertes en éthanol et acétaldéhyde éventuellement entrainés avec le solvant d'extraction, a fortiori ne mentionne pas l'acidification de l'eau de contre-extraction et encore moins des moyens pour limiter la consommation d'acide tout en limitant les pertes en éthanol et acétaldéhyde.

Le procédé selon l'invention vise à limiter ce problème de consommation d'acide. Plus généralement, la présente invention vise la purification d'une charge hydroalcoolique comprenant de l'éthanol et de l'acétaldéhyde, tout en maximisant l'élimination des impuretés, en particulier peu ou pas polaires, contenues dans cette charge aqueuse et limitant les pertes en éthanol et en acétaldéhyde. Lorsqu'elle est avantageusement intégrée à un procédé de type Lebedev, tel que celui décrit dans le brevet FR 3 026 100 ou le brevet FR 3 026 101, la présente invention a pour objectif de purifier l'effluent liquide issu de l'étape de séparation du butadiène en sortie des réacteurs, et plus particulièrement de l'effluent éthanol / acétaldéhyde / eau issu de D1) du brevet FR 3 026 100 ou de l'effluent éthanol / acétaldéhyde / eau issu de l'étape B) du brevet FR 3 026 101, en maximisant l'élimination des impuretés, en particulier peu ou non polaires (comme le diéthyléther) contenues dans ledit effluent liquide tout en optimisant les quantités d'éthanol et d'acétaldéhyde non convertis et recyclés vers les réacteurs, et ce en minimisant la consommation d'acide. La présente invention permet ainsi l'amélioration du rendement global du procédé Lebedev de conversion de l'éthanol en butadiène, avec une consommation en acide réduite.

### Résumé de l'invention

L'invention concerne un procédé purification d'une charge hydroalcoolique (1) comprenant au moins de l'eau, de l'éthanol, de l'acétaldéhyde et des impuretés, ledit procédé comprenant :
a) une étape d'extraction liquide-liquide à contre-courant, comprenant une section d'extraction comprenant un extracteur (2) alimenté en tête par ladite charge hydroalcoolique (1) et au moins une fraction d'un raffinat intermédiaire issu de l'étape b) de contre-extraction et en fond par un solvant d'extraction (3), et produisant en tête un flux d'extraction (5) et en fond un raffinat (4) comprenant de l'eau, de l'éthanol et de l'acétaldéhyde, ladite section d'extraction étant opérée à une température moyenne dans l'extracteur comprise entre 15 et 30°C ;
b) une étape de contre-extraction liquide-liquide à contre-courant comprenant une section de contre-extraction comprenant un contre-extracteur (6) distinct de l'extracteur de l'étape a) et alimenté en tête par une solution aqueuse acide (7), ayant un pH compris entre 0,5 et 5,0, et en fond par le flux d'extraction (5) issu de l'étape a), et produisant en tête un extrait (8) et en fond ledit raffinat intermédiaire, ladite section de contre-extraction étant opérée à une température moyenne dans le contre-extracteur distincte de la température moyenne dans l'extracteur de l'étape a) et comprise entre 40 et 80°C.

L'invention concerne un enchainement d'opérations unitaires pour l'extraction des impuretés et la décomposition du diéthylacétal et/ou hémiéthylacétal contenue dans une charge composé d'eau, d'éthanol, d'acétaldéhyde et d'impuretés, en particulier une charge de type Lebedev.

De manière surprenante, la demanderesse a découvert qu'en imposant un système particulier d'extraction / contre-extraction liquide-liquide à deux colonnes distinctes et en contrôlant un certain nombre de paramètres opératoires telles que les températures d'extraction et de contre-extraction, en plus du pH de la solution aqueuse utilisée pour réaliser l'étape de réextraction, l'extraction des impuretés, en particulier peu ou non polaires de la charge hydroalcoolique est maximisée, la réextraction de diéthylacétal et de l'hémiéthylacétal optimisée et donc les pertes en éthanol et acétaldéhyde limitées, avec une consommation réduite en acide utilisé pour acidifier le solvant aqueux de contre-extraction.

Avantageusement, le procédé selon l'invention est intégré dans un procédé Lebedev (ou de type Lebedev), c'est-à-dire un procédé de conversion d'éthanol en butadiène, notamment en 1,3-butadiène. Lorsqu'il est avantageusement intégré à un procédé de type Lebedev, tel que celui décrit dans le brevet FR 3 026 100 ou FR 3 026 101, le procédé selon l'invention permet ainsi de purifier efficacement par une méthode relativement simple d'extraction/contre-extraction liquide-liquide l'effluent hydroalcoolique obtenu après séparation du butadiène de l'effluent directement issu des réacteurs de conversion, tout en réduisant les pertes en réactifs non convertis et donc d'améliorer les performances du procédé de production de butadiène à partir d'éthanol, tout en en diminuant les coûts opératoires.

### Description des modes de réalisation

Selon l'invention, le composé 1,1-diéthoxyéthane, aussi appelé acétal diéthylique, éthylidène diéthyl éther ou diéthylacétal de l'acétaldéhyde, est dénommé, dans la présente description, diéthylacétal ou DEA. Il peut être défini, selon la présente invention, comme une forme condensée de l'éthanol avec l'acétaldéhyde. L'hémiacétal correspondant est le 1-éthoxyéthan-1-ol ou hémiéthylacétal de l'acétaldéhyde et est appelé, dans la présente description, hémiéthylacétal ou HEA.

Selon la présente invention, la « température moyenne » dans la section d'extraction ou la section de contre-extraction est une température calculée selon la moyenne arithmétique d'au moins deux valeurs de température données par des thermocouples répartis de manière régulière tout au long de l'extracteur (ou du contre-extracteur) ou la température déterminée à l'aide d'un thermocouple situé au centre de l'extracteur (ou du contre-extracteur). Par exemple, dans le cas où il y a deux thermocouples pour déterminer la température moyenne dans la colonne d'extraction, un thermocouple est placé dans la moitié supérieure de la colonne le deuxième dans la moitié inférieure, et de préférence de sorte que le thermocouple de la partie supérieure de la colonne se trouve à une distance par rapport à la tête de la colonne d'extraction égale à celle entre le thermocouple de la partie inférieure et le fond de la colonne d'extraction. Plus le nombre de thermocouples disposés régulièrement tout au long de l'extracteur (ou du contre-extracteur) est grand, plus la température moyenne dans l'extracteur (ou dans le contre-extracteur) est précise.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

L'invention concerne un procédé de purification d'une charge hydroalcoolique comprenant au moins de l'eau, de l'éthanol, de l'acétaldéhyde et des impuretés, ledit procédé comprenant, de préférence consistant en, les étapes suivantes :
a) une étape d'extraction liquide-liquide à contre-courant, comprenant une section d'extraction comprenant un extracteur (2) alimenté en tête par ladite charge hydroalcoolique (1) et au moins une fraction d'un raffinat intermédiaire issu de l'étape b) de contre-extraction et en fond par un solvant d'extraction (3), et produisant en tête un flux d'extraction (5) et en fond un raffinat (4) comprenant de l'eau, de l'éthanol et de l'acétaldéhyde, ladite section d'extraction étant opérée à une température moyenne dans l'extracteur comprise entre 15 et 30°C ;
b) une étape de contre-extraction liquide-liquide à contre-courant comprenant une section de contre-extraction comprenant un contre-extracteur (6) distinct de l'extracteur de l'étape a) et alimenté en tête par une solution aqueuse acide (7), ayant un pH compris entre 0,5 et 5,0, et en fond par le flux d'extraction (5) issu de l'étape a), et produisant en tête un extrait (8) et en fond ledit raffinat intermédiaire, ladite section de contre-extraction étant opérée à une température moyenne dans le contre-extracteur distincte à la température moyenne dans l'extracteur de l'étape a) et comprise entre 40 et 80°C.

### La charge hydroalcoolique :

Le procédé selon l'invention permet d'extraire l'éthanol et l'acétaldéhyde d'une charge hydroalcoolique comprenant de l'eau, de l'éthanol et de l'acétaldéhyde. Ladite charge hydroalcoolique comprend également des impuretés, notamment organiques, qui peuvent être de natures très variées, par exemple des hydrocarbures saturés, insaturés, aromatiques, produits oxygénés, parmi lesquels on peut citer les alcools, cétones, aldéhydes, composés phénoliques, acides, esters, éthers, la masse molaire des différentes impuretés pouvant aller de 50 à 10 000 g/mol. Typiquement, les impuretés peuvent être l'acétone, le diéthyléther, le butanal, des butanols, des butanones, l'éthyle acétate, le crotonadélhyde, des pentènes, des pentadiènes, des hexènes et des hexadiènes.

La charge hydroalcoolique dans le procédé selon l'invention peut éventuellement également comprendre au moins un acétal et/ou hémiacétal. En particulier, la charge hydroalcoolique à traiter peut comprendre du diéthylacétal et/ou l'hémiéthylacétal. Le diéthylacétal et l'hémiéthylacétal sont connu pour être les produits de la réaction de l'éthanol avec l'acétaldéhyde.

Selon l'invention, le terme « impuretés » désigne ainsi tout composé, notamment organique, autre que l'eau, l'éthanol et l'acétaldéhyde et autre que les acétals et hémiacétals, en particulier autre que le diéthylacétal et l'hémiéthylacétal. Les impuretés peuvent, par exemple, être des hydrocarbures saturés, insaturés, aromatiques, comme des pentènes, des pentadiènes, des hexènes et des hexadiènes, ou des produits oxygénés, comme l'acétone, le diéthyléther, le butanal, des butanols, des butanones, l'éthyle acétate, le crotonadélhyde.

En particulier, certaines des impuretés peuvent être considérées comme peu ou pas polaires, lorsqu'elles présentent avec un coefficient de partage, en particulier massique, entre la phase organique d'extraction et la phase aqueuse d'extraction de préférence supérieur ou égal à 1, préférentiellement supérieur ou égal à 2.

Le procédé de purification selon l'invention est ainsi avantageusement alimenté par une charge hydroalcoolique comprenant au moins de l'eau, de l'éthanol, de l'acétaldéhyde, des impuretés et éventuellement des acétals et/ou hémiacétals, en particulier du diéthylacétal et/ de l'hémiéthylacétal.

De préférence, la charge hydroalcoolique comprend entre 30 % et 70% poids d'éthanol, de préférence entre 40 et 60% poids d'éthanol, par rapport au poids total de la charge hydroalcoolique, entre 1 et 30% poids d'acétaldéhyde, de préférence entre 5 et 10% poids d'acétaldéhyde, par rapport au poids total de la charge hydroalcoolique, et entre 0,5 et 20% poids d'impuretés, notamment entre 1 et 20% poids d'impuretés par rapport au poids total de la charge hydroalcoolique. Lorsque la charge comprend, en outre, au moins un acétal et/ou hémiacétal, comme le diéthylacétal et/ou hémiéthylacétal, la teneur pondérale en acétals et hémiacétals dans ladite charge hydroalcoolique est de préférence comprise entre 1 et 20% poids, et de manière préférée entre 1 et 15% poids.

Avantageusement, ladite charge hydroalcoolique est issue de la conversion de l'éthanol en butadiène, en particulier en 1,3-butadiène, après séparation des incondensables et du butadiène. Ladite charge hydroalcoolique est de manière préférée un effluent hydroalcoolique issu d'une étape de séparation du butadiène en sortie des réacteurs de conversion dans un procédé Lebedev, par exemple un effluent semblable à l'effluent éthanol/acétaldéhyde/eau issu de D1) du brevet FR 3 026 100 ou l'effluent éthanol/acétaldéhyde/eau issu de l'étape B) du brevet FR 3 026 101.

### Etape a) d'extraction des impuretés :

Conformément à l'invention, le procédé de purification selon l'invention comprend une étape a) d'extraction liquide-liquide à contre-courant comprenant une section d'extraction comprenant un extracteur (2) alimenté en tête par ladite charge hydroalcoolique (1) et au moins une fraction du raffinat intermédiaire issu de l'étape b) de contre-extraction et en fond par un solvant d'extraction (3), et produisant en tête un flux d'extraction (5), appelé aussi extrait intermédiaire, et en fond un raffinat (4) comprenant de l'eau, l'éthanol et de l'acétaldéhyde, appelé encore raffinat hydroalcoolique.

Selon l'invention, ladite section d'extraction est opérée à une température moyenne dans l'extracteur comprise entre 15 et 30°C. Si la température moyenne dans l'extracteur est inférieure à 10°C ou supérieure à 40°C, l'extraction des impuretés, en particulier peu ou pas polaires est moins efficace.

Le solvant d'extraction et la charge hydroalcoolique qui alimentent ladite section d'extraction de l'étape a) sont avantageusement chacun indépendamment à une température d'entrée comprise entre 10 et 40°C.

Avantageusement, la pression de ladite section d'extraction de l'étape a) est ajustée de manière à ce que les différents flux traversant ladite section restent sous forme liquide. De préférence, ladite section d'extraction de l'étape a) est opérée à une pression comprise entre 0,1 et 0,5 MPa, préférentiellement entre 0,2 et 0,4 MPa.

Le temps de séjour dans la section d'extraction de ladite étape a) est avantageusement ajusté de manière à obtenir les performances désirées en terme de taux de récupération, comme il est connu de l'Homme du métier. En particulier, le temps de séjour dans la section d'extraction de ladite étape a) est compris entre 0,5 et 10,0 h, préférentiellement entre 0,5 et 8,0 h, de manière préférée entre 1,0 et 6,0 h. Le temps de séjour dans la section d'extraction est défini comme un temps de séjour de la phase aqueuse et correspond au ratio entre le volume total occupé par la phase aqueuse considérée dans la section d'extraction par rapport au débit volumique de cette phase aqueuse en sortie de section d'extraction.

Selon l'invention, le solvant d'extraction qui alimente l'étape a) est avantageusement un solvant organique, de préférence apolaire. De préférence, le solvant d'extraction qui alimente l'étape a) est un mélange d'hydrocarbures ayant entre 6 et 40 atomes de carbones, de préférence entre 10 et 20 atomes de carbone ou tout autre solvant permettant une démixtion avec la phase hydro-alcoolique. De manière non limitative, ledit mélange d'hydrocarbures peut être une coupe gazole ou kérosène désulfurée ou bien encore une coupe d'hydrocarbures produite par une unité de type Fischer-Tropsh. De manière préférée, le solvant d'extraction qui alimente l'étape a) est l'hexadécane.

Il est bien connu de l'Homme du métier qu'une extraction liquide-liquide, en particulier à contre-courant, fonctionne avec deux phases liquides, l'une des phases constituant la phase continue et l'autre constituant la phase dispersée, présente sous forme de gouttes distinctes. La nature continue ou dispersée dépend du débit relatif d'une phase par rapport à l'autre. Selon le phénomène bien connu, la nature de la phase dispersée et de la phase continue dépend des débits relatifs de ces phases. Ainsi, si l'on réduit le débit de la phase continue en augmentant le débit de la phase dispersée, la phase dispersée deviendra continue et inversement.

Avantageusement, la section d'extraction est opérée avec un rapport de débit massique de phase continue par rapport au débit massique de phase dispersée inférieur à 70, de préférence inférieure à 35, de manière préférée inférieure à 10, et de préférence inférieur à 3, de préférence inférieur à 1,5. Au-delà de 70, le fonctionnement hydrodynamique de la section d'extraction est compromis. Peu importe que le solvant d'extraction (phase organique) forme la phase continue ou dispersée, ce critère étant un critère hydrodynamique. De manière préférée, dans l'étape a) d'extraction, la phase continue est la phase organique et la phase dispersée est la phase aqueuse.

Plus le rapport de débit massique de solvant d'extraction (c'est-à-dire débit massique du solvant d'extraction entrant dans la section d'extraction) par rapport au débit massique de l'alimentation de ladite section d'extraction de ladite étape a), composée de la charge hydroalcoolique et d'au moins une fraction du raffinat intermédiaire issu de l'étape b) de contre-extraction, est élevé, plus l'étape a) d'extraction des impuretés est efficace. Cependant, un ratio élevé des débits conduit à extraire également une fraction importante d'éthanol et d'acétaldéhyde dans le flux d'extraction produit en tête de la section d'extraction de l'étape a), et par conséquent à augmenter le débit de solution aqueuse nécessaire lors de l'étape b) pour limiter les pertes en éthanol et acétaldéhyde. La valeur du rapport de débit massique de solvant d'extraction sur le débit massique de charge aqueuse doit donc être ajustée de manière à extraire le maximum d'impuretés, notamment pas ou peu polaires, tout en limitant les pertes en éthanol et acétaldéhyde. Le rapport du débit massique de solvant d'extraction par rapport au débit massique de charge d'alimentation de l'étape a), composée de la charge hydroalcoolique et d'au moins une fraction du raffinat intermédiaire issu de l'étape b), est de préférence compris entre 0,1 et 5,0, préférentiellement entre 0,2 et 2,0, de manière préférée entre 0,3 et 1,0.

Le débit massique de solvant d'extraction ainsi que le débit massique de solution aqueuse acide (c'est-à-dire le débit massique de solvant de contre extraction) alimentant l'étape b), sont avantageusement ajustés de manière à ce que l'extrait produit lors de l'étape b) comprenne au moins 50% poids, de préférence au moins 60% poids, de manière préférée au moins 70% poids, de manière très préférée au moins 80% poids, voire au moins 90% poids, des impuretés peu ou pas polaires contenues dans la charge hydroalcoolique alimentant ladite étape a) du procédé selon l'invention, et au plus 5% poids, de préférence au plus 2% poids, et de manière préférée au plus 1% poids de la quantité pondérale totale d'éthanol et d'acétaldéhyde (sous forme libre et/ou condensé, par exemple sous forme de diéthylacétal et/ou hémiéthylacétal) contenue dans ladite charge hydroalcoolique alimentant ladite étape a). De préférence, le rapport du débit massique de la solution aqueuse acide (c'est-à-dire le débit massique de solvant de contre-extraction) alimentant l'étape b) par rapport au débit massique de solvant d'extraction alimentant l'étape a) est compris entre 0,1 et 5,0, de préférence entre 0,2 et 2,0, préférentiellement entre 0,3 et 1,0, de manière préférée entre 0,4 et 0,5.

Le contact entre les deux phases liquides dans ladite section d'extraction de l'étape a) est avantageusement réalisé au sein d'un extracteur. Différentes technologies d'extracteur peuvent être envisagées : l'extracteur peut, par exemple, être une colonne garnie, une colonne pulsée, une colonne compartimentée agitée, compartimentée à l'aide de plateaux perforés, de disques ou de couronnes, ou bien encore une batterie de mélangeurs-décanteurs ou de mélangeurs-centrifugeuses. De préférence, l'extracteur est une colonne pulsée, une colonne compartimentée agitée. Avantageusement, l'extracteur, utilisé dans l'étape a) d'extraction, comprend entre 1 et 20, préférentiellement entre 2 et 8 étages théoriques d'extraction.

L'extrait intermédiaire (ou flux d'extraction) (5), qui est produit en tête de ladite section d'extraction de l'étape a) du procédé selon l'invention, comprend avantageusement au moins 50% poids, de préférence au moins 60% poids, de manière préférée au moins 70% poids et de manière très préférée au moins 80% poids des impuretés peu ou pas polaires de la charge hydroalcoolique. Ledit extrait intermédiaire (5) comprend de l'éthanol et de l'acétaldéhyde présents dans la charge hydroalcoolique qui alimente le procédé selon l'invention, sous leur forme libre et/ou condensée. Ainsi, l'extrait intermédiaire (5) produit en tête de section d'extraction dans l'étape a) comprend très probablement du diéthylacétal et/ou de l'hémiéthylacétal, éventuellement compris dans la charge hydroalcoolique alimentant le procédé selon l'invention, et/ou formés par réaction de l'éthanol avec l'acétaldéhyde lors du procédé selon l'invention en particulier dans ladite section d'extraction dans l'étape a).

Le raffinat (4) hydroalcoolique produit en fond de la section d'extraction de ladite étape a) comprend de l'eau et entre 80 et 100% poids de la quantité totale d'éthanol et acétaldéhyde contenue dans ladite charge hydroalcoolique alimentant le procédé selon l'invention. En d'autres termes, entre 80 et 100% poids de la quantité totale d'éthanol et acétaldéhyde de la charge hydroalcoolique alimentant le procédé selon l'invention sont récupérés dans le raffinat (4) hydroalcoolique produit en fond de la section d'extraction de l'étape a) du procédé selon l'invention.

L'étape a) du procédé selon l'invention est configurée de manière à extraire le maximum d'impuretés, en particulier peu ou pas polaires (comme de diéthyl éther), et le minimum d'éthanol et d'acétaldéhyde (sous forme libre et/ou condensée).

Dans une telle configuration, les acétals et/ou hémiacétals, en particulier le diéthylacétal et/ou hémiéthylacétal, éventuellement présents dans la charge hydroalcoolique et/ou formés au cours du procédé selon l'invention, notamment lors de l'étape a) d'extraction, sont extraits de manière importante par le solvant d'extraction dans le flux d'extraction, avec les impuretés en particulier peu ou pas polaires au cours de l'étape a) d'extraction, dans la mesure où lesdits acétals et/ou hémiacétals sont beaucoup moins polaires que l'éthanol et l'acétaldéhyde libres. Le flux d'extraction (5) extrait en tête de la section d'extraction de ladite étape a) alimente l'étape b) de contre-extraction.

### Etape b) de contre-extraction :

Conformément à l'invention, le procédé de purification comprend une étape b) de contre-extraction liquide-liquide à contre-courant, dont l'objectif est de contre-extraire les composés nobles, c'est-à-dire l'éthanol et l'acétaldéhyde sous forme libre et/ou condensée en hémi- ou di-éthylacétal, de manière efficace.

Selon l'invention, l'étape b) de contre-extraction liquide-liquide à contre-courant comprend une section de contre-extraction comprenant un contre-extracteur (6) distinct de l'extracteur (2) de l'étape a), alimenté en tête par une solution aqueuse acide (7), ayant un pH compris entre 0,5 et 5,0, et en fond par le flux d'extraction (5) issu de l'étape a), et produisant en tête un extrait (8) et en fond un raffinat intermédiaire.

La section de contre-extraction est, selon l'invention, opérée à une température moyenne dans le contre-extracteur distincte de la température moyenne dans l'extracteur de l'étape a) et comprise entre 40 et 80°C, de préférence comprise entre 45 et 70°C, préférentiellement entre 45°C et 60°C. La section d'extraction dans l'étape a) est opérée à une température moyenne dans l'extracteur entre 15 et 30°C et de manière très préférée, la section de contre-extraction dans l'étape b) est opérée à une température moyenne dans le contre-extracteur entre 45 et 70°C, en particulier entre 45 et 60°C. Pour des valeurs de température moyenne dans la section de contre-extraction supérieures à 80°C, il y a, en plus d'une augmentation du cout de production générée par une augmentation de la consommation énergétique nécessaire à la chauffe, un risque de formation de bulles dans les phases aqueuse et organique, entrant une perte en efficacité de contre-extraction. Lorsque la température moyenne dans la section de contre-extraction est inférieure à 40°C, l'efficacité de contre-extraction apparait non optimale. Ladite solution aqueuse acide et le flux d'extraction issu de l'étape a) alimentent avantageusement ladite section de contre-extraction de l'étape b) chacun indépendamment à une température d'entrée comprise entre 10 et 90°C, de préférence entre 40 et 90°C.

Selon un mode de réalisation particulier de l'invention, la section de contre-extraction comprend une colonne adiabatique (dite isolée), comme contre-extracteur, alimentée en tête par la solution aqueuse acide à une température d'entrée de ladite solution aqueuse acide dans ladite colonne adiabatique comprise entre 50 et 90°C, de préférence entre 60 et 85°C, et en fond par le flux d'extraction issu de l'étape a). La température d'entrée dans ladite colonne adiabatique du flux d'extraction issu de l'étape a) est fixée par la température à laquelle est opérée de l'étape a) d'extraction, soit en moyenne entre 15°C et 30°C. Dans ce mode de réalisation particulier de l'invention, un gradient de température générée entre la tête de la colonne et le fond permet d'obtenir une température moyenne dans la colonne adiabatique de contre-extraction distincte de la température moyenne dans la section d'extraction de l'étape a), suffisamment élevée, avantageusement compris entre 40 et 80°C, pour obtenir les effets recherchés, c'est-à-dire une réextraction optimisée de l'éthanol et acétaldéhyde et/ou des diacétals et hémiacétals contenus dans le flux d'extraction issu de l'étape a).

Avantageusement, la pression dans ladite section de contre-extraction est ajustée de manière à ce que les différents flux traversant ladite section restent sous forme liquide. De préférence, la section de contre-extraction de l'étape b) est opérée à une pression comprise entre 0,1 et 0,5 MPa, préférentiellement entre 0,2 et 0,4 MPa.

Avantageusement, la section de contre-extraction de l'étape b) est opérée avec un temps de séjour, plus précisément un temps de séjour en phase aqueuse, dans la section de contre-extraction compris entre 0,5 et 10,0 h, préférentiellement entre 0,5 et 8,0 h, de manière préférée entre 1 et 6,0 h. Ledit temps de séjour dans la section de contre-extraction est défini comme le temps moyen nécessaire à une molécule d'eau injectée avec la solution aqueuse acide alimentant ladite section de contre-extraction de ladite étape b) pour être extraite dans le raffinat intermédiaire issu de ladite section de contre-extraction de ladite étape b). Ce temps de séjour est classiquement déterminé par mesure de DTS, ou Distribution de Temps de Séjour, dans laquelle un marqueur (colorant ou autre), est injecté ponctuellement en entrée, la concentration en marqueur étant observée en sortie.

Selon l'invention, le solvant de contre-extraction utilisé dans l'étape b) est une solution aqueuse acide ayant un pH entre 0,5 et 5,0, de préférence entre 2 et 4,0, et de manière préférée entre 2,5 et 3,5. Ladite solution aqueuse acide qui alimente l'étape b) est avantageusement de l'eau acidifiée par ajout d'un composé acide de sorte que le pH de la solution aqueuse est compris entre 0,5 et 5,0, de préférence entre 2 et 4,0, et de manière préférée entre 2,5 et 3,5. La solution aqueuse acide peut ainsi contenir de manière non limitative des acides forts et/ou des acides faibles. De manière non limitative, l'eau est acidifiée avec un acide faible comme de l'acide acétique, ou un acide fort comme l'acide sulfurique ou l'acide nitrique, de préférence avec l'acide acétique. De manière préférée, la solution aqueuse acide est de l'eau acidifiée avec de l'acide acétique, de sorte que ladite solution aqueuse acide comprend une teneur en acide acétique inférieure à 3% poids d'acide acétique, de préférence inférieure ou égale à 3,0% poids, de manière préférée inférieure ou égale à 1,5% poids par rapport au poids total de ladite solution aqueuse acide.

En outre, ladite solution aqueuse acide qui alimente l'étape b) contient de préférence moins de 2% poids, de manière préférée moins de 1% poids, de la quantité totale (éthanol+acétaldéhyde) par rapport au poids total de ladite solution aqueuse acide, c'est-à-dire que la somme des teneurs massiques en éthanol et acétaldéhyde dans ladite solution aqueuse acide est inférieure à 2% poids, de préférence inférieure à 1% poids par rapport au poids total de ladite solution aqueuse acide. De manière très préférée, ladite solution aqueuse acide ne contient ni éthanol, ni acétaldéhyde.

Le contact entre les deux phases liquides, la phase organique et la phase aqueuse, dans ladite section de contre-extraction de l'étape b) est avantageusement réalisé au sein d'un contre-extracteur. Différentes technologies pour ledit contre-extracteur peuvent être envisagées : colonne garnie, colonne pulsée, colonne compartimentée agitée, compartimentée à l'aide de plateaux perforés, de disques ou de couronnes, ou bien encore une batterie de mélangeurs-décanteurs ou de mélangeurs-centrifugeuses. De préférence, le contre-extracteur est une colonne pulsée, une colonne compartimentée agitée. Avantageusement, le contre-extracteur, utilisé dans l'étape b) de contre-extraction, comprend entre 1 et 20, préférentiellement entre 1 et 5 étages théoriques.

Avantageusement, les débits massiques de la phase aqueuse et la phase organique dans la section de contre-extraction sont ajustés de sorte que le ratio (Q_{aq. entrant} / Q_{orga. sortant}) du débit massique de la phase aqueuse entrant dans la section de contre-extraction (Q_{aq. entrant}) par rapport au débit de la phase organique sortant de la section de contre-extraction (Q_{orga. sortant}) est de préférence entre 0,1 et 5,0, préférentiellement entre 0,2 et 2,0, de manière préférée entre 0,3 et 1,0.

De préférence, l'étape b) est opérée de sorte que la phase aqueuse constitue la phase continue dans la section de contre-extraction et la phase organique la phase dispersée dans ladite section de contre-extraction de ladite étape b).

De manière très avantageuse, la phase aqueuse constitue la phase dispersée dans la section d'extraction de l'étape a) et la phase continue dans la section de contre-extraction de l'étape b).

Dans de telles conditions opératoires, les acétals et/ou hémiacétals, en particulier le diéthyl acétal et/ou hémiéthylacétal, éventuellement présents dans la charge hydroalcoolique et/ou formés au cours du procédé selon l'invention notamment par réaction entre l'éthanol et l'acétaldéhyde, se décomposent en particulier en éthanol et acétaldéhyde, qui peuvent être réextraits dans la phase aqueuse de la section de contre-extraction, de préférence la phase continue de la section de contre-extraction, permettant ainsi d'améliorer la récupération des pertes en éthanol et acétaldéhyde. Cette décomposition permet ainsi, lorsque le procédé de purification selon l'invention est intégré à un procédé Lebedev, de limiter les pertes en réactifs et, par conséquent, d'améliorer le rendement en butadiène du procédé Lebedev global.

L'étape b) de contre-extraction du procédé selon l'invention produit, en tête de la section de contre-extraction, un extrait (8) comprenant avantageusement au moins 50% poids, de préférence au moins 60% poids, de manière préférée au moins 70% poids, de manière très préférée au moins 80% poids, voire au moins 90% poids, des impuretés, en particulier peu ou pas polaires, de la charge hydroalcoolique alimentant l'étape a) du procédé selon l'invention. L'extrait (8) produit en tête de la section de contre-extraction de l'étape b) comprend avantageusement moins de 1,0% poids, de préférence moins de 0,1% poids, de manière préférée moins de 0,001% poids d'acétals et/ou hémiacétals, en particulier de diéthylacétal et/ou d'hémiéthylacétal. De manière très préférée, l'extrait produit en tête de la section de contre-extraction de l'étape b) est exempt d'acétals et/ou hémiacétals, en particulier exempt de diéthylacétal et/ou d'hémiéthylacétal.

L'extrait issu de l'étape b) peut ensuite être traité par exemple dans une étape de purification/séparation afin de récupérer le solvant d'extraction pour pouvoir le recycler vers l'étape a) d'extraction.

La section de contre-extraction de l'étape b) produit en fond un raffinat intermédiaire comprenant avantageusement de l'eau, de l'éthanol et de l'acétaldéhyde. Au moins une fraction dudit raffinat intermédiaire, avantageusement la totalité dudit raffinat intermédiaire, est introduite en tête de la section d'extraction de l'étape a). Même lorsque la totalité du raffinat intermédiaire est introduite dans la section d'extraction, un soutirage peut avantageusement être réalisé de manière continue ou discontinue sur ledit raffinat intermédiaire, ledit soutirage étant appelé purge, afin de limiter l'accumulation des impuretés dans ce raffinat intermédiaire.

De manière surprenante, la demanderesse a découvert qu'en opérant l'extraction (ou lavage) et la contre-extraction (ou contre-lavage) dans deux extracteurs distincts, en utilisant une solution aqueuse acide comme solvant de contre-extraction et en imposant deux températures distinctes dans les deux colonnes, l'élimination des impuretés en particulier peu ou pas polaires de la charge hydralcoolique à traiter, par exemple de l'effluent liquide issu des réacteurs de conversion de l'éthanol en butadiène, comme dans un procédé Lebedev, était très efficace tandis que les pertes en éthanol et acétaldéhyde étaient de manière surprenante limitées et la consommation en acide, nécessaire pour acidifier l'eau de contre-extraction, réduite sensiblement.

Avantageusement, le procédé selon l'invention permet d'atteindre une efficacité d'extraction des impuretés peu ou pas polaire, en particulier une efficacité d'extraction du diéthyléther, supérieure ou égale à 75% poids, de préférence supérieure ou égale à 80% poids et une efficacité de contre-extraction, en particulier du diéthylacétal, forme condensée de l'éthanol et de l'acétaldéhyde, supérieure ou égale à 90% poids, de préférence supérieure ou égale à 95% poids et de manière préférée supérieure ou égale à 97% poids.

Par « efficacité d'extraction », on entend selon l'invention l'efficacité d'extraction du diéthyléther, définie par le rapport (Q_{DEE extrait} / Q_{DEE entrant}) entre le débit massique de diéthyléther (DEE) sortant du procédé, c'est-à-dire présent dans l'extrait obtenu en tête de la section de contre-extraction(Q_{DEE extrait}), sur le débit massique de diéthyléther (DEE) entrant dans la section d'extraction, c'est-à-dire présent dans la charge hydroalcoolique alimentant la section d'extraction (Q_{DEE entrant}). Plus particulièrement, l'efficacité d'extraction du diéthyéther (DEE) est calculée de la manière suivante : Efficacité d'extraction DEE = [(teneur massique en DEE de l'extrait sortant en tête de la section de contre-extraction) x (débit massique de l'extrait sortant en tête de la section de contre-extraction) / (teneur massique en DEE de la charge hydroalcoolique) x (débit massique de la charge hydroalcoolique)].

Par « efficacité de contre-extraction », on entend l'efficacité d'extraction du diéthylacétal, définie par le rapport entre la quantité massique de diéthylacétal contre-extraite de l'extrait intermédiaire et la quantité massique de diéthylacétal présent dans l'extrait intermédiaire entrant dans la section de contre-extraction. Plus particulièrement, l'efficacité de contre-extraction du diéthylacétal (DEA) est calculée de la manière suivante : Efficacité de contre-extraction DEA = 1 - [(teneur massique en DEA de l'extrait sortant en tête de la section de contre-extraction) x (débit massique de l'extrait sortant en tête de la section de contre-extraction) / (teneur massique en DEA de l'extrait intermédiaire entrant dans la section de contre-extraction) x (débit massique de l'extrait intermédiaire entrant dans la section de contre-extraction)].

Les teneurs massiques en DEE et en DEA des différents flux considérés pour calculer les efficacités d'extraction et de contre-extraction sont déterminées par toute méthode connue de l'Homme du métier, par exemple par chromatographie en phase gazeuse.

Le procédé de purification selon l'invention peut avantageusement être intégré dans un procédé global de type Lebedev, c'est-à-dire un procédé global de conversion de l'éthanol en butadiène, comme étape de purification de l'effluent liquide hydroalcoolique issu d'une étape de séparation du butadiène en sortie des réacteurs de conversion de l'éthanol en butadiène. Dans un procédé de type Lebedev, l'effluent liquide hydroalcoolique issu de l'étape de séparation en sortie des réacteurs de conversion de l'éthanol en butadiène comprend en effet de l'eau, provenant notamment de la conversion de l'éthanol en butadiène, et des réactifs, l'éthanol et acétaldéhyde, non convertis ou convertis seulement partiellement. Ledit effluent liquide hydroalcoolique comprend également des impuretés constituées de molécules organiques, comme l'acétone, le diéthyléther, le butanal, des butanols, des butanones, l'éthyle acétate, le crotonadélhyde, des pentènes, des pentadiènes, des hexènes ou des hexadiènes, et éventuellement des acétals et/ou hémiacétals, notamment du diéthylacétal et/ou hémiéthylacétal. Lorsque le procédé de traitement selon l'invention est intégré dans un procédé de type Lebedev, le taux de recyclage des composés réactifs, c'est-à-dire l'éthanol et l'acétaldéhyde, est amélioré, permettant ainsi une optimisation du rendement global en butadiène du procédé Lebedev, et ceci sans engendrer de surcoût qui pourrait être dû à une consommation de composé acide ou de post-traitement de la phase aqueuse.

Les Figures intégrées à la présente description et les exemples qui suivent sont présentés à titre illustratif et non limitatif du procédé de purification selon l'invention.

### Liste des figures

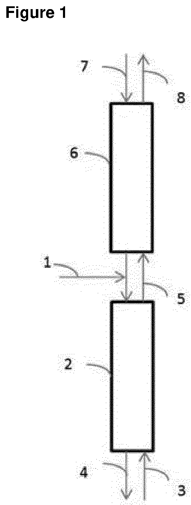

La Figure 1 représente de manière schématique et non limitative un arrangement du procédé selon l'invention. La charge hydroalcoolique comprenant de l'eau, de l'éthanol, de l'acétaldéhyde et des impuretés (1) alimente en tête une section d'extraction liquide-liquide (2) dans laquelle est mise en oeuvre l'étape a). La section d'extraction liquide-liquide (2) est également alimentée en tête par un raffinat intermédiaire issu de la section de contre-extraction (6) de l'étape b), et en fond par un solvant d'extraction (3). Un flux d'extraction (5), encore appelé extrait intermédiaire, est produit en tête de la section d'extraction (2) tandis qu'un raffinat (4) est soutiré en fond de section d'extraction (2). Le flux d'extraction (5) alimente, en fond, la section de contre-extraction (6) de l'étape b). La section de contre-extraction (6) est alimentée, en tête, par une solution aqueuse acide (7). Un extrait (8) est soutiré en tête de la section de contre-extraction (6) tandis qu'en fond un raffinat intermédiaire est produit. Ledit raffinat intermédiaire alimente la section d'extraction liquide-liquide (2).

### Exemples

Dans les exemples suivants, une charge hydroalcoolique comprenant de l'éthanol, de l'acétaldéhyde, de l'eau et des impuretés en particulier peu ou pas polaires, comme le diéthyléther (considéré comme impureté), de composition donnée dans le tableau 1 et de 3,65 kg/heure de débit massique, est traitée.

| **Composés** | **Teneur pondérale (% poids par** |
|---|---|
| | **rapport au poids total de la charge)** |
| Acétaldéhyde | 3,91% |
| Ethanol | 62,95% |
| Acétone | 0,07% |
| Ethyl vinyl ether | 0,04% |
| Diethylether | 1,00% |
| Butanal | 0,03% |
| butanone | 0,01% |
| Ethyl acetate | 1,37% |
| Acide acétique | 0,63% |
| Butanol | 0,41% |
| Diéthylacétal | 4,46% |
| Styrène | 0,10% |
| Eau | 25,01% |

Dans les exemples suivants, les sections d'extraction et contre-extraction sont opérées dans les mêmes conditions suivantes, seules diffèrent les températures moyennes des sections d'extraction et de contre-extraction et la teneur massique en acide acétique de la solution aqueuse acide utilisée comme solvant de contre-extraction.

Les colonnes d'extraction et de contre-extraction sont des colonnes distinctes, toutes les deux de type ECR Sulzer avec des internes ayant des ouvertures de plateaux de 40%, de 32 mm de diamètre interne et de hauteur utile 1,8 m.

La colonne d'extraction est alimentée en fond en hexadécane à un débit de 1,28 kg/heure. La colonne de contre-extraction est alimentée en tête par une eau acidifiée par de l'acide acétique, le débit de ce solvant de contre-extraction étant à 0,56 kg/heure.

La colonne d'extraction est opérée en phase aqueuse dispersée. La colonne de contre-extraction est opérée en phase aqueuse continue.

La vitesse d'agitation est adaptée dans les deux colonnes de sorte que la fraction volumique en phase dispersée soit environ de 15% volumique dans la colonne d'extraction et 3% volumique dans la colonne de contre-extraction.

La colonne d'extraction produit en tête un flux d'extraction qui est injecté en fond de colonne de contre-extraction. La colonne d'extraction produit en fond un raffinat comprenant de l'éthanol et de l'acétaldéhyde. La colonne de contre-extraction produit en tête de colonne un extrait et en fond un raffinat intermédiaire, ce dernier étant injecté en tête de colonne d'extraction.

Dans les différents exemples, la concentration en acide acétique de la solution aqueuse acide (solvant de contre-extraction), donc le pH de cette solution aqueuse acide, ainsi que les températures moyennes des sections d'extraction et contre-extraction varient. Le Tableau 2 ci-dessous récapitule les conditions opératoires variables et les résultats obtenus en termes d'efficacité d'extraction de l'impureté diéthyl éther et d'efficacité de contre-extraction du diéthylacétal.

L'efficacité d'extraction du diéthyl éther (DEE) et l'efficacité de contre-extraction du diéthylacétal (DEA) sont calculées, pour chaque exemple, comme présenté ci-avant dans la description, c'est-à-dire de la manière suivante : Efficacité d'extraction du DEE = [(teneur massique en DEE de l'extrait) x (débit massique de l'extrait) / (teneur massique en DEE de la charge hydroalcoolique) x (débit massique de la charge hydroalcoolique)] Efficacité de contre-extraction DEA = 1 - [(teneur massique en DEA de l'extrait) x (débit massique de l'extrait) / (teneur massique en DEA de l'extrait intermédiaire) x (débit massique de l'extrait intermédiaire)].

Les teneurs massiques en DEE de l'extrait et de la charge hydroalcoolique et les teneurs massiques en DEA de l'extrait et de l'extrait intermédiaire ont été déterminées par chromatographie en phase gazeuse.

| **Exemples** | **Teneur en acide acétique (% poids)** | **pH de la solution aqueuse acide** | **Températu re d'extractio n (°C)** | **Températu re de contre-extraction (°C)** | **Efficacité d'extraction du DEE (% poids)** | **Efficacité de contre-extraction du DEA (% poids)** |
|---|---|---|---|---|---|---|
| 1 | 3% | 2,5 | 20 | 20 | 81% | 93% |
| 2 | 1% | 2,8 | 20 | 20 | 81% | 74% |
| 3 | 1% | 2,8 | 40 | 40 | 72% | 92% |
| 4 | 1% | 2,8 | 50 | 50 | 43% | 98% |
| 5 | 1% | 2,8 | 20 | 50 | 81% | 98% |

L'exemple 1 illustre le cas de référence conformément à l'art antérieur. Avec une teneur en acide acétique du solvant de contre-extraction de 3% poids et une température d'opération égale à 20°C pour les deux colonnes, l'efficacité d'extraction du diéthyléther (DEE) et l'efficacité de contre-extraction du diéthylacétal (DEA) sont satisfaisantes, puisque respectivement supérieure à 80% poids (81% d'efficacité d'extraction du DEE) et supérieure à 90% poids (93% d'efficacité de contre-extraction du DEA).

L'exemple 2 montre que, à même température de fonctionnement que précédemment (20°C dans les deux colonnes), lorsque la teneur en acide acétique dans la solution aqueuse acide utilisée comme solvant de contre-extraction diminue (1% poids à la place de 3% poids), l'efficacité de contre-extraction du DEA diminue sensiblement et devient inférieure à 75% poids.

D'après les exemples 3 et 4, la hausse conjointe de la température dans les deux colonnes (extraction et contre-extraction), à 40°C pour l'exemple 3 et 50°C pour l'exemple 4, permet de retrouver une efficacité de contre-extraction satisfaisante (respectivement 92% et 98%) pour des teneurs en acide acétique dans le solvant aqueux de contre-extraction faible (1%). Cependant, l'effet sur l'extraction du DEE est néfaste puisque l'efficacité d'extraction du DEE devient inférieure à 75% : plus précisément l'efficacité d'extraction du DEE est de 72% poids dans l'exemple 3 et de 43% poids dans l'exemple 4.

L'exemple 5, conforme à l'invention, démontre clairement que, même pour une teneur faible en acide acétique dans la solution aqueuse acide utilisée comme solvant de contre-extraction, le fait d'imposer deux températures distinctes entre l'extraction et la contre-extraction, et en particulier une température de 20°C dans la colonne d'extraction et une température moyenne de 50°C dans la colonne de contre-extraction, permet d'obtenir une extraction du DEE optimisée, avec une efficacité d'extraction du DEE égale à 81% poids, et une contre-extraction du DEA élevée, avec une efficacité de contre-extraction du DEA égale à 98% poids, efficacité de contre-extraction qui est supérieure à celle obtenue dans l'exemple 1 selon l'art antérieur.

Il apparait ainsi clairement que le procédé selon l'invention, de purification d'une charge hydroalcoolique comprenant de l'eau, de l'éthanol, de l'acétaldéhyde et des impuretés, permet d'extraire les impuretés peu ou pas polaire de manière optimale et d'améliorer la contre-extraction de la forme condensée de l'éthanol et de l'acétaldéhyde, avec une consommation en acide acétique sensiblement réduite.

## Revendications

1. Procédé de purification d'une charge hydroalcoolique (1) comprenant au moins de l'eau, de l'éthanol, de l'acétaldéhyde et des impuretés, ledit procédé comprenant :
a) une étape d'extraction liquide-liquide à contre-courant, comprenant une section d'extraction comprenant un extracteur (2) alimenté en tête par ladite charge hydroalcoolique (1) et au moins une fraction d'un raffinat intermédiaire issu de l'étape b) de contre-extraction et en fond par un solvant d'extraction (3), et produisant en tête un flux d'extraction (5) et en fond un raffinat (4) comprenant de l'eau, de l'éthanol et de l'acétaldéhyde, ladite section d'extraction étant opérée à une température moyenne dans l'extracteur comprise entre 15 et 30°C ;
b) une étape de contre-extraction liquide-liquide à contre-courant comprenant une section de contre-extraction comprenant un contre-extracteur (6) distinct de l'extracteur de l'étape a) et alimenté en tête par une solution aqueuse acide (7), ayant un pH compris entre 0,5 et 5,0, et en fond par le flux d'extraction (5) issu de l'étape a), et produisant en tête un extrait (8) et en fond ledit raffinat intermédiaire, ladite section de contre-extraction étant opérée à une température moyenne dans le contre-extracteur distincte de la température moyenne dans l'extracteur de l'étape a) et comprise entre 40 et 80°C.

2. Procédé selon la revendication 1 dans lequel la température moyenne dans la colonne de contre-extraction (6) de l'étape b) est comprise entre 45 et 60°C.

3. Procédé selon l'une des revendications précédentes, dans lequel le contre-extracteur de l'étape b) est une colonne adiabatique alimentée en tête par ladite solution aqueuse acide (7), à une température d'entrée de ladite solution aqueuse acide dans ladite colonne adiabatique comprise entre 50 et 90°C, de préférence entre 60 et 85°C.

4. Procédé selon l'une des revendications précédentes, dans lequel ladite solution aqueuse acide (7) qui alimente la colonne de contre-extraction (6) de l'étape b) présente un pH compris entre 2 et 4, de préférence entre 2,5 et 3,5.

5. Procédé selon l'une des revendications précédentes, dans lequel ladite solution aqueuse acide (7) est de l'eau acidifiée avec de l'acide acétique de sorte que ladite solution aqueuse acide comprend une teneur en acide acétique inférieure à 3% poids, de manière préférée inférieure ou égale à 1,5% poids d'acide acétique.

6. Procédé selon l'une des revendications précédentes, dans lequel la charge hydroalcoolique (1) comprend entre 30 % et 70% poids d'éthanol, de préférence entre 40 et 60% poids d'éthanol, par rapport au poids total de la charge hydroalcoolique, entre 1 et 30% poids d'acétaldéhyde, de préférence entre 5 et 10% poids d'acétaldéhyde, par rapport au poids total de la charge hydroalcoolique, et entre 0,5 et 20% poids d'impuretés, notamment entre 1 et 20% poids d'impuretés par rapport au poids total de la charge hydroalcoolique.

7. Procédé selon l'une des revendications précédentes, dans lequel la charge hydroalcoolique (1) comprend, en outre, au moins un acétal et/ou hémiacétal, en particulier du diéthylacétal et/ou de l'hémiéthylacétal.

8. Procédé selon l'une des revendications précédentes, dans lequel la charge hydroalcoolique (1) est un effluent hydroalcoolique issu d'une étape de séparation du butadiène en sortie des réacteurs de conversion dans un procédé Lebedev.

9. Procédé selon l'une des revendications précédentes, dans lequel le solvant d'extraction (3) est un solvant organique, de préférence apolaire, de manière préférée un mélange d'hydrocarbures ayant entre 6 et 40 atomes de carbones, de préférence entre 10 et 20 atomes de carbone.

10. Procédé selon la revendication 9, dans lequel le solvant d'extraction (4) est l'hexadécane.

11. Procédé selon l'une des revendications précédentes, dans lequel ladite solution aqueuse acide (7) qui alimente la colonne de contre-extraction (6) de l'étape b) comprend moins de 2% poids, de préférence moins de 1% poids, de l'ensemble éthanol et acétaldéhyde, de manière préférée est exempte d'éthanol et d'acétaldéhyde.

## Patentansprüche

1. Verfahren zur Reinigung eines hydroalkoholischen Einsatzmaterials (1), das mindestens Wasser, Ethanol, Acetaldehyd und Verunreinigungen umfasst, das Verfahren umfassend:
a) einen Schritt der Flüssig-Flüssig-Extraktion im Gegenstrom, umfassend einen Extraktionsabschnitt, der einen Extraktor (2) umfasst, dem im Kopf das hydroalkoholische Einsatzmaterial (1) und mindestens eine Fraktion eines aus dem Schritt b) zur Rückextraktion hervorgegangenen Zwischenraffinats und im Sumpf ein Extraktionslösungsmittel (3) zugeführt werden und der im Kopf einen Extraktionsstrom (5) und im Sumpf ein Raffinat (4), das Wasser, Ethanol und Acetaldehyd umfasst, erzeugt, wobei der Extraktionsabschnitt bei einer mittleren Temperatur in dem Extraktor zwischen 15 und 30 °C betrieben wird;
b) einen Schritt der Flüssig-Flüssig-Rückextraktion im Gegenstrom, umfassend einen Rückextraktionsabschnitt, der einen Rückextraktor (6) umfasst, der von dem Extraktor des Schritts a) verschieden ist und dem im Kopf eine saure wässrige Lösung (7) mit einem pH-Wert zwischen 0,5 und 5,0 und im Sumpf der aus dem Schritt a) hervorgegangene Extraktionsstrom (5) zugeführt werden und der im Kopf ein Extrakt (8) und im Sumpf das Zwischenraffinat erzeugt, wobei der Rückextraktionsabschnitt bei einer mittleren Temperatur in dem Rückextraktor betrieben wird, die verschieden von der mittleren Temperatur in dem Extraktor des Schritts a) ist und zwischen 40 und 80 °C beträgt.

2. Verfahren nach Anspruch 1, wobei die mittlere Temperatur in der Rückextraktionskolonne (6) des Schritts b) zwischen 45 und 60 °C beträgt.

3. Verfahren nach einem der vorhergehenden Ansprüche, wobei der Rückextraktor des Schritts b) eine adiabate Kolonne ist, der im Kopf die saure wässrige Lösung (7) mit einer Einlasstemperatur der sauren wässrigen Lösung in die adiabate Kolonne zwischen 50 und 90 °C, bevorzugt zwischen 60 und 85 °C, zugeführt wird.

4. Verfahren nach einem der vorhergehenden Ansprüche, wobei die saure wässrige Lösung (7), die der Rückextraktionskolonne (6) des Schritts b) zugeführt wird, einen pH-Wert zwischen 2 und 4, bevorzugt zwischen 2,5 und 3,5, aufweist.

5. Verfahren nach einem der vorhergehenden Ansprüche, wobei die saure wässrige Lösung (7) mit Essigsäure angesäuertes Wasser ist, so dass die saure wässrige Lösung einen Essigsäuregehalt kleiner 3 Gew.-%, bevorzugt kleiner oder gleich 1,5 Gew.-% Essigsäure, umfasst.

6. Verfahren nach einem der vorhergehenden Ansprüche, wobei das hydroalkoholische Einsatzmaterial (1) zwischen 30 Gew.-% und 70 Gew.-% Ethanol, bevorzugt zwischen 40 und 60 Gew.-% Ethanol, bezogen auf das Gesamtgewicht des hydroalkoholischen Einsatzmaterials, zwischen 1 und 30 Gew.-% Acetaldehyd, bevorzugt zwischen 5 und 10 Gew.-% Acetaldehyd, bezogen auf das Gesamtgewicht des hydroalkoholischen Einsatzmaterials, und zwischen 0,5 und 20 Gew.-% Verunreinigungen, insbesondere zwischen 1 und 20 Gew.-% Verunreinigungen bezogen auf das Gesamtgewicht des hydroalkoholischen Einsatzmaterials umfasst.

7. Verfahren nach einem der vorhergehenden Ansprüche, wobei das hydroalkoholische Einsatzmaterial (1) ferner mindestens ein Acetal und/oder Halbacetal, insbesondere Diethylacetal und/oder Hemiethylacetal, umfasst.

8. Verfahren nach einem der vorhergehenden Ansprüche, wobei das hydroalkoholische Einsatzmaterial (1) ein hydroalkoholischer Austrag ist, der aus einem Schritt zur Trennung des Butadiens im Auslass der Konversionsreaktoren in einem Lebedev-Verfahren hervorgegangen ist.

9. Verfahren nach einem der vorhergehenden Ansprüche, wobei das Extraktionslösungsmittel (3) ein organisches, bevorzugt apolares, Lösungsmittel ist, vorzugsweise ein Gemisch aus Kohlenwasserstoffen, die zwischen 6 und 40 Kohlenstoffatome, bevorzugt zwischen 10 und 20 Kohlenstoffatome besitzen.

10. Verfahren nach Anspruch 9, wobei das Extraktionslösungsmittel (4) Hexadecan ist.

11. Verfahren nach einem der vorhergehenden Ansprüche, wobei die saure wässrige Lösung (7), die der Rückextraktionskolonne (6) des Schritts b) zugeführt wird, weniger als 2 Gew.-%, bevorzugt weniger als 1 Gew.-%, der Menge von Ethanol und Acetaldehyd umfasst, bevorzugt frei von Ethanol und Acetaldehyd ist.

## Claims

1. Process for the purification of an aqueous/alcoholic feedstock (1) comprising at least water, ethanol, acetaldehyde and impurities, said process comprising:
a) a step of countercurrentwise liquid-liquid extraction, comprising an extraction section comprising an extractor (2) fed at the top by said aqueous/alcoholic feedstock (1) and at least a fraction of an intermediate raffinate resulting from the back-extraction step b) and at the bottom by an extraction solvent (3), and producing at the top an extraction stream (5) and at the bottom a raffinate (4) comprising water, ethanol and acetaldehyde, said extraction section being operated at a mean temperature in the extractor of between 15 and 30°C;
b) a step of countercurrentwise liquid-liquid back-extraction comprising a back-extraction section comprising a back-extractor (6) distinct from the extractor of step a) and fed at the top by an acidic aqueous solution (7), having a pH of between 0.5 and 5.0, and at the bottom by the extraction stream (5) resulting from step a), and producing at the top an extract (8) and at the bottom said intermediate raffinate, said back-extraction section being operated at a mean temperature in the back-extractor distinct from the mean temperature in the extractor of step a) and of between 40 and 80°C.

2. Process according to Claim 1, in which the mean temperature in the back-extraction column (6) of step b) is of between 45 and 60°C.

3. Process according to either of the preceding claims, in which the back-extractor of step b) is an adiabatic column fed at the top by said acidic aqueous solution (7), at a temperature for entry of said acidic aqueous solution into said adiabatic column of between 50 and 90°C, preferably between 60 and 85°C.

4. Process according to one of the preceding claims, in which said acidic aqueous solution (7) which feeds the back-extraction column (6) of step b) exhibits a pH of between 2 and 4, preferably between 2.5 and 3.5.

5. Process according to one of the preceding claims, in which said acidic aqueous solution (7) is water acidified with acetic acid, so that said acidic aqueous solution comprises a content of acetic acid of less than 3% by weight, preferably of less than or equal to 1.5% by weight, of acetic acid.

6. Process according to one of the preceding claims, in which the aqueous/alcoholic feedstock (1) comprises between 30% and 70% by weight of ethanol, preferably between 40% and 60% by weight of ethanol, with respect to the total weight of the aqueous/alcoholic feedstock, between 1% and 30% by weight of acetaldehyde, preferably between 5% and 10% by weight of acetaldehyde, with respect to the total weight of the aqueous/alcoholic feedstock, and between 0.5% and 20% by weight of impurities, in particular between 1% and 20% by weight of impurities, with respect to the total weight of the aqueous/alcoholic feedstock.

7. Process according to one of the preceding claims, in which the aqueous/alcoholic feedstock (1) additionally comprises at least one acetal and/or hemiacetal, in particular diethyl acetal and/or ethyl hemiacetal.

8. Process according to one of the preceding claims, in which the aqueous/alcoholic feedstock (1) is an aqueous/alcoholic effluent resulting from a step of separation of the butadiene at the outlet of the conversion reactors in a Lebedev process.

9. Process according to one of the preceding claims, in which the extraction solvent (3) is an organic solvent, preferably a nonpolar organic solvent, in a preferred way a mixture of hydrocarbons having between 6 and 40 carbon atoms, preferably between 10 and 20 carbon atoms.

10. Process according to Claim 9, in which the extraction solvent (4) is hexadecane.

11. Process according to one of the preceding claims, in which said acidic aqueous solution (7) which feeds the back-extraction column (6) of step b) comprises less than 2% by weight, preferably less than 1% by weight, of the ethanol and acetaldehyde combination, in a preferred way is devoid of ethanol and acetaldehyde.
